(19) Europäisches Patentamt
European Patent Office
Office européen des brevets

(11) Veröffentlichungsnummer: **0 321 966 A1**

(12) # EUROPÄISCHE PATENTANMELDUNG

(21) Anmeldenummer: **88121440.7**

(51) Int. Cl.⁴: **C07D 277/40**

(22) Anmeldetag: **21.12.88**

(30) Priorität: **23.12.87 CH 5015/87**

(43) Veröffentlichungstag der Anmeldung: **28.06.89 Patentblatt 89/26**

(84) Benannte Vertragsstaaten: **CH DE FR GB IT LI**

(71) Anmelder: **LONZA AG**
**Gampel/Wallis Geschäftsleitung Basel**
**CH-4002 Basel(CH)**

(72) Erfinder: **Huwiler, Alfred, Dr.**
**Sennjistrasse**
**Baltschieder (Kanton Wallis)(CH)**
Erfinder: **Abächerli, Claudio, Dr.**
**Sandstrasse 5**
**Visp (Kanton Wallis)(CH)**
Erfinder: **Squaratti, Armand**
**Chemin des Bousses**
**Granges (Kanton Wallis)(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**D-8000 München 40(DE)**

(54) **Thioester und Verfahren zu deren Herstellung.**

(57) Es werden neue Thioester der Formel I beschrieben, die als wertvolle Zwischenprodukte zur Herstellung von Cephalosporinen mit breitem antimikrobiellen Wirkungsspektrum dienen.

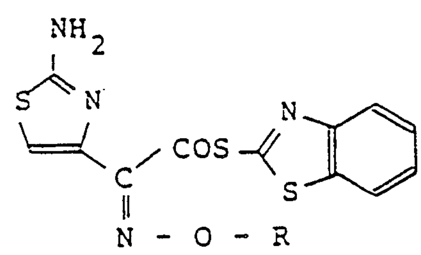

(I)

worin R Allyl oder Cyclopentyl bedeutet.

EP 0 321 966 A1

## Thioester und Verfahren zu deren Herstellung

Die Erfindung betrifft neue Aminothiazolylessigsäurebenzthiazolthioester der Formel

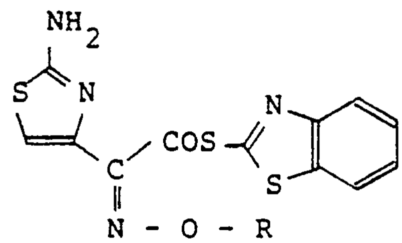

I

worin R Allyl oder Cyclopentyl bedeutet sowie ein Verfahren zur Herstellung dieser Verbindungen.

Die genannten neuen Thioester sind wichtige Zwischenprodukte zur Herstellung von Cephalosporinen mit breitem antimikrobiellen Wirkungsspektrum, wie sie u.a. in der CH-PS 650 786 und der JP Anm. 61 24592 beschrieben sind.

Die Herstellung der neuen Thioester nach Patentanspruch 1 erfolgt erfindungsgemäss nach Patentanspruch 2, indem man bei Aminothiazolessigsäureestern der Formel

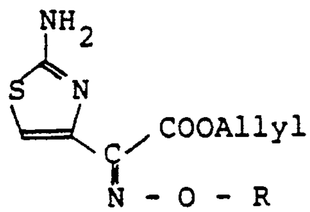

II

worin R die genannte Bedeutung hat, in Gegenwart einer Palladiumverbindung, eines Phosphins oder Phosphits und einer Base die Allylgruppe abspaltet und das entstandene Salz in einer zweiten Stufe mit Dithio-bis-benzthiazol, einem Phosphin oder Phosphit und gegebenenfalls einer Base, zum Endprodukt umsetzt.

Die Verbindungen der Formel II sind, ausgehend von Diketen, durch Ueberführung mit Chlor und Allylalkohol in den 4-Chloracetessigsäureallylester, Weiterüberführung mit Thioharnstoff zum Aminothiazolhydroxyiminoessigsäureallylester oder durch Umesterung von Ethyl-2-(2-aminothiazol-4-yl)-2-hydroxyiminoessigsäure mit Allylalkohol in Gegenwart von Natriumallylat zum Aminothiazolhydroxyiminoessigsäureallylester und schliesslich Umsetzung mit Allyl- bzw. Cyclopentylbromid, einfach herstellbar.

Als Palladiumverbindungen kommen z.B. Palladiumkohle, Palladiumsalze, insbesondere Pd-halogenide oder -acetate oder auch Palladium-organische Komplexverbindungen zusammen mit den eingesetzten Phosphiten oder Phosphinen in Betracht. Besonders geeignet ist Palladium-II-acetat.

Die Palladiumverbindungen gelangen zweckmässig in katalytischen Mengen von 0,01 bis 1 Mol%, bezogen auf den eingesetzten Aminothiazolallylester, zum Einsatz.

Zur Abspaltung der Allylgruppe ist zusätzlich ein Phosphit oder Phosphin, wie z.B. ein Tri($C_1$-$C_2$)-alkylphosphit, bevorzugt Triethylphosphit oder Triphenylphosphin, einzusetzen.

Das entsprechende Phosphit oder Phosphin wird zweckmässig in katalytischen Mengen zwischen 0,5 bis 10 Mol%, bezogen auf den Aminothiazolallylester, verwendet.

Weiterer Reaktionsteilnehmer ist eine organische Base. Zweckmässige Vertreter sind die tertiären Amine, wie Triethylamin, N-Methylmorpholin oder N-Methylpyrrolidin. Besonders geeignet ist N-Methylmorpholin oder N-Methylpyrrolidin.

Die Menge organischer Base wird so gewählt, dass zumindest die notwendige Menge für die Bildung des entsprechenden Aminothiazolacetats zur Verfügung steht.

Die Reaktionstemperatur bewegt sich in der Regel zwischen 0 und 40 °C.

Das entstandene Salz wird zweckmässig isoliert, bevor es mit Dithio-bis-benzthiazol, einem Phosphit oder Phosphin und gegebenenfalls einer Base weiterumgesetzt wird.

Es ist aber durchaus möglich, ohne Isolierung des entstandenen Salzes in situ auf genannte weise zum Endprodukt umzusetzen.

Der ersten Stufe entsprechend werden für die zweite Stufe aus der Reihe der Phosphine oder Phosphite vorteilhaft die Tri($C_1$-$C_2$)alkylphosphite, besonders bevorzugt Triethylphosphit oder Triphenylphosphine, angewendet.

Bezogen auf 1 Mol des entstandenen Aminothiazolacetats wird das Phosphit bzw. das Phosphin zweckmässig in stöchiometri schen Mengen, vorteilhaft in Mengen zwischen 1 und 1,5 Mol, eingesetzt.

Als Basen werden zweckmässig organische Basen, wie tertiäre Amine, eingesetzt.

Bevorzugte Vertreter sind entsprechend der vorhergehenden Stufe N-Methylmorpholin, N-Methylpyrrolidin oder Triethylamin. Zweckmässig werden beide Stufen des Verfahrens in einem organischen Lösungsmittel, vorteilhaft beide Stufen im gleichen Lösungsmittel, durchgeführt. Besonders geeignet sind Acetonitril, Aceton, Methylenchlorid oder andere aprotische Lösungsmittel. Die Reaktionstemperatur für die letzte Stufe liegt zweckmässig zwischen -20 und +40 °C.

Nach einer Reaktionszeit von 15 Minuten bis 5 Stunden kann das Reaktionsgemisch auf übliche Weise aufgearbeitet und der gewünschte Thioester abgetrennt werden.

Beispiel 1

Allyl-2-(2-aminothiazol-4-yl)-2-hydroxyiminoacetat (syn-Isomeres)

In 905 ml Allylalkohol wurden 9,5 g (0,41 Mol) Natriummetall aufgelöst. In diese Lösung wurden 90 g Ethyl-2-(2-aminothiazol-4-yl)-2-hydroxyiminoacetat (syn-Isomeres) (0,41 Mol) zugegeben und 2 Stunden gerührt. Danach wurde das Reaktionsgemisch unter vermindertem Druck eingeengt. Anschliessend wurden 900 ml Toluol zugegeben und erneut unter vermindertem Druck eingeengt. Die Aufschlämmung wurde abgenutscht, mit Toluol gewaschen und getrocknet. 85 g des Allyl-2-(2-aminothiazol-4-yl)-2-hydroxyiminoacetat-Natriumsalzes wurden in 850 ml Eiswasser mit 10%iger Salzsäure auf pH 7 gestellt, abgenutscht, mit Wasser gewaschen und getrocknet. Es wurden 75 g (= 98%) Allyl-2-(2-aminothiazol-4-yl)-2-hydroxyiminoacetat (syn-Isomeres) erhalten.

NMR: (DMSO-$d_6$) $\delta$ in ppm
4,75 (d, 2H)
5,25 (d, 1H)
5,94 (d, 1H)
5,89-6,02 (m, 1H)
6,85 (s, 1H)
7,19 (s, 2H)
11,7 (s, 1H)

Beispiel 2

Allyl-2-(2-aminothiazol-4-yl)-2-allyloxyiminoacetat (syn-Isomeres)

13,63 g (60 mMol) Allyl-2-(2-aminothiazol-4-yl)-2-hydroxyiminoacetat (syn-Isomeres) wurden in 42 ml N,N-Dimethylformamid vorgelegt, auf -15 °C gekühlt und mit 5,26 g Natronlauge (49,5%) versetzt. Danach wurden bei -10 bis 0 °C 7,87 g (65,1 mMol) Allylbromid in einer Stunde zugegeben und 3 Stunden bei dieser Temperatur weitergerührt. Zum Ansatz wurden nun 120 ml Wasser zugetropft und das ausgefallene Produkt abgenutscht, mit Wasser gewaschen und unter vermindertem Druck getrocknet. Man isolierte 14,47 g Allyl-2-(2-aminothiazol-4-yl)-2-allyl oxyiminoacetat (syn-Isomeres), entsprechend einer Ausbeute von 90%.

NMR: (DMSO-$d_6$) $\delta$ in ppm
4,63 (d, 2H)
4,79 (d, 2H)
5,20-5,45 (m, 4H)
5,87-6,03 (m, 2H)
6,92 (s, 1H)
7,28 (s breit, 2H)

IR: (KBr) $cm^{-1}$ 3425, 3258, 3129, 1718, 1616, 1538
Anstelle des freien Allyl-2-(2-aminothiazol-4-yl)-2-hydroxyiminoacetates kann als Edukt auch direkt das Allyl-2-(2-aminothiazol-4-yl)-2-hydroxyiminoacetat-Natriumsalz aus dem Beispiel 1 eingesetzt werden.

Beispiel 3

N-Allyl-N-methylpyrrolidinium-2-(2-aminothiazol-4-yl)-2-allyloxyiminoacetat (syn-Isomeres)

5,67 g (21,2 mMol) Allyl-2-(2-aminothiazol-4-yl)-2-allyloxyiminoacetat (syn-Isomeres) und 3,6 mg Palladium-II-acetat wurden in 60 ml Acetonitril vorgelegt und 0,20 g Triethylphosphit bei Raumtemperatur zugetropft. Danach wurden 1,77 g (20,8 mMol) N-Methylpyrrolidin in 15 Minuten zudosiert. Es wurde zwei Stunden bei Raumtemperatur weitergerührt, auf 0 °C abgekühlt und das ausgefallene Salz abgenutscht, mit Acetonitril gewaschen und getrocknet, Man isolierte so 6,17 g (= 82,5%) N-Allyl-N-methylpyrrolidinium-2-(2-aminothiazol-4-yl)-2-allyloxyiminoacetat.
NMR: (DMSO-$d_6$) δ in ppm
2,00-2,14 (s breit, 4H)
2,98 (s, 3H)
3,35-3,55 (m, 4H)
4,05 (d, 2H)
4,43 (d, 2H)
5,12-5,20 (m, 1H)
5,28-5,40 (m, 1H)
5,59-5,72 (m, 2H)
5,88-6,16 (m, 2H)
6,54 (s, 1H)
7,02 (s breit, 2H)
IR: (KBr) $cm^{-1}$ 3400 breit, 3200, 1609, 1536, 1393

Beispiel 4

2-Mercaptobenzthiazolyl-2-(2-aminothiazol-4-yl)-2-allyloxyiminoacetat (syn-Isomeres)

1,84 g (5, 22 mMol) N-Allyl-N-methylpyrrolidinium-2-(2-aminothiazol-4-yl)-2-allyloxyiminoacetat (syn-Isomeres) wurden zusammen mit 0,49 g N-Methylmorpholin, 2,04 g (6,15 mMol) 2,2′-Dithiobisbenzothiazol in 15 ml Acetonitril vorgelegt. Danach wurden 1,17 g (7,05 mMol) Triethylphosphit in einer Stunde bei -5 °C zudosiert. Nach weiteren 30 Minuten Rühren wurde das Produkt abgenutscht mit Acetonitril gewaschen und getrocknet. Dabei isolierte man 1,40 g (=71,2%)2-Mercaptobenzothiazolyl-2-(2-aminothiazol-4-yl)-2-allyloxyiminoacetat (syn Isomeres).
NMR: (DMSO-$d_6$) δ in ppm
4,72 (d, 2H)
5,23-5,40 (m, 2H)
5,93-6,07 (m, 1H)
7,06 (s, 1H)
7,41 (s breit, 2H)
7,53-7,65 (m, 2H)
8,09 (d, 1H)
8,23 (d, 1H)
IR: (KBr) $cm^{-1}$ 3321, 3141, 1698, 1652, 1540
Die Reaktion kann auch in situ ohne Isolation des N-Allyl-N-methylpyrrolidinium-2-(2-aminothiazol-4-yl)-2-allyloxyiminoacetates aus der vorhergehenden Stufe (Beispiel 3) durchgeführt werden.

Beispiel 5

Allyl-2-(2-aminothiazol-4-yl)-2-cyclopentyloxyiminoacetat (syn-Isomeres)

13,63 g (60 mMol) Allyl-2-(2-aminothiazol-4-yl)-2-hydroxyiminoacetat (syn-Isomeres) wurden in 51 ml N,N-Dimethylformamid und 3,7 ml Wasser vorgelegt. Dann wurden 16,58 g (120 mMol) Kaliumcarbonat eingerührt. Zur Suspension wurden 8,94 g 60 mMol) Bromcyclopentan bei Raumtemperatur zugetropft und der Ansatz auf 50 °C aufgeheizt. Man rührte 6 Stunden bei dieser Temperatur, kühlte auf Raumtemperatur und fällte das Produkt durch Zugabe von 105 ml Wasser aus. Danach wurde abgenutscht, mit Wasser gewaschen und getrocknet. Man isolierte 11,92 g (= 67%) Allyl-2-(2-aminothiazol-4-yl)-2-cyclopentyl oxyiminoacetat (syn-Isomeres).

NMR: (DMSO-$d_6$) δ in ppm
1,45-1,85 (m, 8H)
4,67-4,83 (m, 1H + 2H)
5,23-5,32 (m, 1H)
5,36-5,46 (m, 1H)
5,87-6,02 (m, 1H)
6,92 (s, 1H)
7,29 (s breit, 2H)

Beispiel 6

N-Allyl-N-methylpyrrolidinium-2-(2-aminothiazol-4-yl)-2-cyclopentyloxyiminoacetat (syn-Isomeres)

In analoger Weise, wie in Beispiel 3 beschrieben, wurde ausgehend von Allyl-2-(2-aminothiazol-4-yl)-2-cyclopentyloxyiminoacetat (syn-Isomeres) anstelle des Allyl-2-(2-aminothiazol-4-yl)-2-allyloxyiminoacetates N-Allyl-N-methylpyrrolidinium-2-(2-aminothiazol-4-yl)-2-cyclopentyloxyiminoacetat (syn-Isomeres) in einer Ausbeute von 64% isoliert. Dieses Salz kann auch ohne Isolierung direkt für die weitere Synthese in Beispiel 7 verwendet werden.

NMR: (DMSO-$d_6$) δ in ppm
1,40-1,73 (m, 8H)
2,07 (s breit, 4H)
2,97 (s, 3H)
3,35-3,55 (m, 4H)
4,01 (d, 2H)
4,42-4,50 (m, 1H)
5,56-5,68 (m, 2H)
5,98-6,13 (m, 1H)
6,48 (s, 1H)
6,97 (s breit, 2H)
IR: (KBr) $cm^{-1}$ ca. 3400 breit, 3172, 2961, 1607, 1533, 1390

Beispiel 7

2-Mercaptobenzthiazolyl-2-(2-aminothiazol-4-yl)-2-cyclopentyloxyiminoacetat (syn-Isomeres)

Auf analoge Weise, wie in Beispiel 4 beschrieben, wurde ausgehend von N-Allyl-N-methylpyrrolidinium-2-(2-aminothiazol-4-yl)-2-cyclopentyloxyiminoacetat (syn-Isomeres) anstelle des N-Allyl-N-methylpyrrolidinium-2-(2-aminothiazol-4-yl)-2-allyloxyiminoacetates 2-Mercaptobenzothiazolyl-2-(2-aminothiazol-4-yl)-2-cyclopentyloxyiminoacetat (syn-Isomeres) in einer Ausbeute von 70% erhalten.

NMR: (DMSO-$d_6$) δ in ppm
1,50-1,87 (m, 8H)
4,76-4,85 (m, 1H)
7,04 (s, 1H)
7,41 (s breit, 2H)
7,53-7,67 (m, 2H)

8,10 (d, 1H)
8,24 (d, 1H)
IR: (KBr) $cm^{-1}$ 3314, 3147, 2954, 1721, 1646, 1541

**Ansprüche**

1. Thioester der allgemeinen Formel

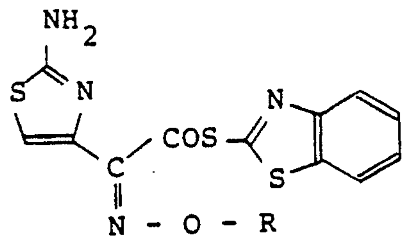

I

worin R Allyl oder Cyclopentyl bedeutet.

2. Verfahren zur Herstellung von Thioestern gemäss Patentanspruch 1, dadurch gekennzeichnet, dass man einem Aminothiazolessigsäureester der Formel

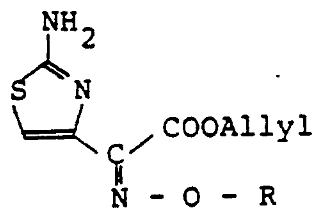

II

worin R die genannte Bedeutung hat, in der ersten Stufe in Gegenwart einer Palladiumverbindung, eines Phosphins oder Phosphits und einer Base die Allylgruppe abspaltet und das entstandene Aminothiazolacetat in der zweiten Stufe mit Dithio-bis-benzthiazol, einem Phosphin oder Phosphit und gegebenenfalls einer Base zum Thioester weiterumsetzt.

3. Verfahren nach Patentanspruch 2, dadurch gekennzeichnet, dass als Palladiumverbindungen Palladiumkohle, Palladiumsalze oder Palladium-organische Verbindungen eingesetzt werden.

4. Verfahren nach Patentanspruch 2 oder 3, dadurch gekennzeichnet, dass als Palladiumverbindung Palladium-II-acetat eingesetzt wird.

5. Verfahren nach einem der Patentansprüche 2 bis 4, dadurch gekennzeichnet, dass in der ersten Stufe aus der Reihe der Phosphine oder Phosphite ein Tri($C_1$-$C_2$)alkylphosphit eingesetzt wird.

6. Verfahren nach einem der Patentansprüche 2 bis 5, dadurch gekennzeichnet, dass als Basen tertiäre Amine, bevorzugt N-Methylmorpholin oder N-Methylpyrrolidin, eingesetzt werden.

7. Verfahren nach einem der Patentansprüche 2 bis 6, dadurch gekennzeichnet, dass in der zweiten Stufe aus der Reihe der Phosphine oder Phosphite ein Tri($C_1$-$C_2$)alkylphosphit oder Triphenylphosphin eingesetzt wird.

Europäisches Patentamt

# EUROPÄISCHER RECHERCHENBERICHT

Nummer der Anmel.

EP 88121440.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A2/A3 - 0 185 221 (F. HOFFMANN-LA ROCHE & CO.)<br>* Zusammenfassung *<br>-- | 1,2 | C 07 D 277/40 |
| P,A | EP - A2 - 0 272 827 (ICI PHARMA)<br>* Seite 15, Formel (A); Seite 16, Formel (5) *<br>-- | 1,2 | |
| A | EP - B1 - 0 096 296 (F. HOFFMANN-LA ROCHE & CO.)<br>* Ansprüche 8,16,19-23 *<br>---- | 1,2,5, 6 | |
| | | | RECHERCHIERTE SACHGEBIETE (Int. Cl.4) |
| | | | C 07 D 277/00 |

Der vorliegende Recherchenbericht wurde für alle Patentansprüche erstellt.

| Recherchenort | Abschlußdatum der Recherche | Prüfer |
|---|---|---|
| WIEN | 27-02-1989 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82